# EUROPÄISCHE PATENTSCHRIFT

(11) **EP 2 844 293 B1**
(45) Veröffentlichungstag und Bekanntmachung des Hinweises auf die Patenterteilung: **22.04.2020**
(21) Anmeldenummer: 13727810.7
(22) Anmeldetag: 06.05.2013
(51) Int. Cl.: A61K 35/02, A61K 31/02, A61K 45/06, A61K 33/00, A61K 33/08, A61K 33/26, A61K 33/42, A61P 1/00

(54) **TONMINERAL ZUR ANWENDUNG IN DER BEHANDLUNG VON CHRONISCH-ENTZÜNDLICHEN DARMERKRANKUNGEN**
CLAY MINERALS FOR USE IN THE TREATMENT OF INFLAMMATORY BOWEL SYNDROMES
ARGILES POUR L'UTILISATION DANS LE TRAITEMENT DES SYNDROMES INFLAMMATOIRES INTESTINAUX

(30) Priorität: 04.05.2012 DE 102012207471
(43) Veröffentlichungstag der Anmeldung: 11.03.2015
(73) Patentinhaber: Fim Biotech GMBH, 10117 Berlin (DE); Fraunhofer-Gesellschaft zur Förderung der angewandten Forschung e.V., 80686 München (DE)
(72) Erfinder: MITZNER, Steffen, 18119 Rostock (DE); KERKHOFF, Claus, 48161 Münster (DE); EMMRICH, Frank, 04299 Leipzig (DE); BREITRÜCK, Anne, 18055 Rostock (DE); BODAMMER, Peggy, 18055 Rostock (DE); KRÜGER, Gerd, 10621 Berlin (DE); DALLWIG, Rainer, 14476 Potsdam (DE)
(74) Vertreter: Maikowski & Ninnemann Patentanwälte Partnerschaft mbB
(86) Internationale Anmeldenummer: PCT/EP2013/059407
(87) Internationale Veröffentlichungsnummer: WO 2013/164491

(56) Entgegenhaltungen:
- WO-A1-2005/112881
- WO-A1-2008/113905
- CN-A- 1 326 746
- RO-A3- 59 608
- Viñals Stein: "Chronisch entzündliche Darmerkrankungen (CED) - Colitis ulcerosa und Morbus Crohn", Naturheilkund und Naturheilverfahren Fachportal , 22. Februar 2010 (2010-02-22), XP002702481, Gefunden im Internet: URL:http://www.heilpraxisnet.de/krankheite n/chronisch-entzuendliche-darmerkrankungen -6567.php [gefunden am 2013-07-12]

## Beschreibung

Die vorliegende Erfindung betrifft ein Wechsellagerungstonmineral zur Verwendung als Mittel zur Prävention und/ Behandlung bei chronisch-entzündlichen Darmerkrankungen (CED) gemäß Anspruch 1, sowie ein mit mindestens einer weiteren Substanz ausgewählt aus der Gruppe der Huminsäuren modifiziertes Wechsellagerungstonmineral gemäß Anspruch 14.

### Beschreibung

Weltweit erkranken jährlich über 4 Milliarden Menschen an Durchfall, wobei über 8 Millionen Menschen an den Folgen des Durchfalls sterben. Die Ursachen für das Auftreten eines Durchfalls bestehen unter anderem in Erkrankungen des Verdauungssystems, Laktoseintoleranz, übermäßige Aufnahme von Koffein und Alkohol, Süßstoffe, Medikamente, Viren, Bakterien, Giftstoffe oder auch durch psychische Belastungen.

Akute Durchfallerkrankungen, verursacht in der Regel durch Infektionen, Lebensmittelvergiftungen oder Medikamente, dauern in der Regel nur wenige Tage an. Dauert hingegen ein Durchfall länger als vier Wochen, so spricht man von chronischem Durchfall. Die häufigste Ursache für chronischen Durchfall ist das Reizdarmsyndrom, aber auch entzündliche Darmerkrankungen, wie Morbus Crohn und Colitis ulcerosa, Infektionen, Nahrungsmittelunverträglichkeiten, Pankreatitis und Zöliakie können zu chronischem Durchfall führen.

Etwa 300.000 Patienten in Deutschland leiden an den CED Morbus Crohn und Colitis ulcerosa. Dabei kommt es zu schubweise verlaufenden Entzündungsreaktionen im Darm verbunden mit schweren Durchfällen, Blutverlust und Schmerzen. Die Patienten müssen dauerhaft medikamentös und/oder chirurgisch behandelt werden. Charakteristisch für Morbus Crohn ist ein diskontinuierlicher, segmentaler Befall der Darmschleimhaut. Dabei können gleichzeitig mehrere Darmabschnitte erkrankt sein, die durch gesunde Abschnitte voneinander getrennt sind. Colitis ulcerosa kennzeichnet sich durch einen entzündlichen Befall des Mastdarms und Dickdarms aus, wobei sich im Falle von Colitis ulcerosa die Entzündung kontinuierlich vom Mastdarm beginnend, also von anal nach oral, ausbreitet, und auf die Darmschleimhaut beschränkt ist.

Der Pathogenesekomplex der CED besteht aus genetischen Faktoren, Umwelteinflüssen, der mikrobiellen Darmflora und der angeborenen Immunität. Es gibt derzeit 5 verschiedene ätiologische Theorien zu der Entstehung der CED: 1. Beanspruchung der Darmwand durch enteroadhärenten und invasiven Bakterienstämme, 2. eine anhaltende Darmdysbiose, 3. eine gestörte Barrierefunktion der Darmschleimhaut, 4. Defekte in der antimikrobiellen Darmbarriere und 5. eine abnormale Immunregulation.

Diese Manifestationen werden ursächlich hervorgerufen durch von außen über den Verdauungstrakt eingetragenen chemischen und biologischen Toxinen, durch Medikamentenmissbrauch hervorgerufene Endotoxine, Infektionen durch das Mycobacterium avium subspecies paratuberculosis oder enteroadhärenten und invasiven *E.coli*-Stämme, falsche Ernährungsweisen und damit auch Unterversorgung der Darmschleimhaut mit essentiellen Nährstoffen, Dysstress und genetische Dispositionen. Diese Multikausalität der CED ist bei jedem Patienten unterschiedlich. Sie münden jedoch immer in einer überschießenden Immunreaktion des gastrointestinalen Immunsystems gegen luminale Antigene im Darm, die letztendlich zu entzündlichen Veränderungen des Darmepithels führen. Da es bisher nicht möglich war, die primären Ursachen der CED zumindest teilweise zu beseitigen, konzentrierte man sich auf die monokausale Behandlung der überschießenden Immunreaktion.

Leitsymptome der CED sind Durchfälle, Blutungen und Bauchschmerzen, wobei es eine Vielzahl individueller Verläufe gibt. Eine sichere klinische Unterscheidung zwischen Morbus Crohn und Colitis ulcerosa ist nicht immer möglich.

Der Krankheitsverlauf der CED kann neben der charakteristischen gastrointestinalen Symptomatik wesentlich durch das Auftreten von extraintestinalen Begleiterkrankungen bestimmt und kompliziert werden.

Gegenwärtig erfolgt die Behandlung von Morbus Crohn und Colitis ulcerosa symptomatisch durch eine "Step-up"-Behandlungsstrategie mittels einer langsamen Eskalation der Medikation hin zu immer stärkerer immunsuppressiver Therapie. Dabei werden solche Medikamente wie Aminosalicylate, Steroide, Immunsuppressiva und Biologika eingesetzt.

Neue therapeutische Ansätze beschäftigen sich mit einer "Top-down"-Therapie, wo mit der derzeit potentesten immunsuppressiven Therapie, den Biologika, begonnen, und bei klinischer Besserung langsam die Intensität der immunsuppressiven Therapie reduziert wird.

Bei einer ausgeprägten Barrierestörung hält die Entzündungsreaktion allerdings an und es ist eine dauerhafte Verabreichung der Medikamente notwendig, die mit erheblichen Nebenwirkungen einhergeht. Ist eine Herabregulierung nicht möglich, bleibt nur noch die Operation mit gravierenden Folgen für die Lebensqualität der Patienten.

Chronischer Durchfall und CED werden ursächlich durch biologische oder chemische Darmtoxine und/oder eine gestörte Darmbarrierefunktion hervorgerufen. Eine Bindung der biologischen und chemischen Toxine könnte zu einer gezielten Unterstützung der Darmflora vorteilhaft sein. Arzneiformen enthaltend Montmorillonit zur Behandlung verschiedener gastrointestinaler Beschwerden sind beispielsweise aus WO2005112881 bekannt.

Die gegenwärtige Standardmedikation gegen die CED Morbus Crohn und Colitis ulcerosa weist eine Reihe von unerwünschten Nebenreaktionen bzw. Komplikationen auf. Zu den typischen Standardmedikamenten gehören beispielhaft Mesalazin, Dexamethason und Mutaflor.

Mesalazin (5-Aminosalicylsäure) ist ein Aminderivat der Salicylsäure und wird als entzündungshemmender Arzneistoff bei der Behandlung von CED angewendet. Dexamethason (9-Fluor-16α-Methylprednisolon) ist ein künstliches Glukokortikoid, welches entzündungshemmend und dämpfend auf das Immunsystem wirkt. Es gehört zu den langwirkenden Glukokortikoiden und wirkt rund 30mal stärker als die körpereigenen Produkte.

Mutaflor ist ein probiotisches Medikament und bisher der einzige natürliche Wirkstoff, der durch verschiedene Wirkmechanismen positiven Einfluss auf das Krankheitsgeschehen der CED durch Ursachenbekämpfung nimmt. Es wird in der Erhaltungstherapie der Colitis ulcerosa bei Unverträglichkeit von Aminosalicylaten eingesetzt. Mutaflor ist ein Konzentrat aus Bakterien von *E*. *coli* Nissle 1917. Es unterstützt dabei die physiologische Wirkung der Darmflora und die Energieversorgung der Schleimhautzellen im Dickdarm. Auch wird das Eindringen von Keimen in die Darmzellen durch Mutaflor gehemmt. Ist der Darm chronisch entzündet, wirkt Mutaflor entzündungshemmend. Nachteilig ist jedoch, dass das Mutaflor aufgrund seiner Zusammensetzung aus *E.coli* Bakterien gekühlt gelagert werden muss, wodurch deren Einsatzmöglichkeiten eingeschränkt sind.

Insbesondere die Aminosalicylate wie z.B. Mesalazin, die Steroide wie z.B. Dexamethason sowie die anderen Immunsuppressiva und Biologika führen bei andauernder und lang anhaltender Einnahme in aufsteigender Reihenfolge zunehmend zu erheblichen Nebenwirkungen.

Aufgabe der vorliegenden Erfindung ist daher die Bereitstellung eines Tonminerals und deren Modifikationen zur Anwendung in therapeutischen Verfahren, insbesondere zur Reduzierung von chronischen Entzündungen im Darmtrakt wie den CED und deren extraintestinalen Manifestationen, wobei auch bei lang anhaltender Einnahme dieser Verbindungen keine wesentlichen Nebenwirkungen auftreten dürfen.

Diese Aufgabe wird durch ein Wechsellagerungstonmineral und deren Modifikationen zur Verwendung als Mittel zur Prävention und/oder Behandlung von chronisch-entzündlichen Darmerkrankungen (CED) gemäß den beigefügten Ansprüchen gelöst.

Entsprechend wird ein Wechsellagerungstonmineral zur Prävention und / oder Behandlung von CED verwendet.

Auch wird gemäß der vorliegenden Erfindung eine mineralische mit mindestens einer weiteren Substanz ausgewählt aus der Gruppe der Huminsäuren modifizierte Verbindung bzw. modifiziertes Wechsellagerungstonmineral Tonmineral zur Prävention und / oder Behandlung von CED verwendet.

Erfindungsgemäß wird das zum Einsatz kommende Wechsellagerungstonmineral vor seiner Verwendung gemahlen wird und einer thermischen Behandlung unterzogen. Bevorzugterweise wird das Tonmineral auf eine Partikelgröße kleiner 5µm, z.B. auf eine Partikelgröße zwischen 1 und 5 µm, insbesondere 1 und 4 µm gemahlen und anschließend thermisch aktiviert. Die thermische Aktivierung (Kalzinierung), die z.B. bei Temperaturen zwischen 300°C und 500°C, bevorzugt 350°C und 450°C z.B. über mehrere Stunden von 1 bis 5 h, bevorzugt 1 bis 3h erfolgt, bewirkt die Bildung von reaktiven Bruchflächen der mineralischen Matrix mit einer erhöhten Bindungsaffinität.

Durch die thermische Behandlung bzw. Kalzinierung wird das Zwischenschichtwasser verdampft und die Struktur wird durch den entweichenden Dampf aufgeweitet. Beide Effekte, die Bildung von Kanälen durch entweichendes Wasser und die Auflockerung der Struktur durch Bruchstücke, bewirken eine bessere Durchdringbarkeit für Adsorptive. Die abgebröckelten Schichten schaffen Eingänge zu den Zwischenschichtbereichen. Dadurch steigen die BET-Oberfläche und das Porenvolumen an. Des Weiteren beobachtet man eine Abnahme des Anteils an Kohlenstoff, der durch Huminstoffe hervorgerufen wird. Insgesamt wird durch die thermische Behandlung eine größere spezifische Oberfläche erzeugt.

Die als Grundbaustein zum Einsatz kommende Wechsellagerungstonmineral verfügt über komplexe Eigenschaften und ermöglicht es, mindestens drei verschiedene Wirkeigenschaften bzw. Wirkrichtungen in einem Produkt zu vereinen und nutzbar zu machen. So ermöglicht die vorliegende mineralische Verbindung die Eliminierung von Toxinen. Dabei erfolgt eine Bindung von Toxinen, wie zum Beispiel Enterotoxinen an spezialisierte Teilchen der Verbindung und führt so zur Vermeidung bzw. Verminderung von Durchfall, hervorgerufen durch pathogene Keime. Darüber hinaus werden Endotoxine, die typischerweise durch Antibiotika vermittelte Reaktionen freigesetzt werden, auf gleiche Weise gebunden. Darüber hinaus erfolgt eine Bindung von sonstigen chemischen und biologischen Schadstoffen, die durch den Verdauungstrakt aufgenommen werden.

Neben der Eliminierung von Toxinen ermöglicht die vorliegende mineralische Verbindung eine Unterstützung der Barrierefunktion des Darms. Bei Verabreichung der mineralischen Verbindung in Form einer kolloid dispersen und gelartigen Suspension kommt es zur Ausbildung einer Schutzschicht über der defekten Mukosabarriere, wodurch das Eindringen von Bakterien und anderen Schadstoffen verhindert wird und dadurch die Entzündungsreaktionen reduziert werden.

Bei Einsatz eines mit einer weiteren Substanz modifizierten Wechsellagerungstonminerals kann ebenfalls die Anzahl pathogener Keime und freier Radikale verringert werden. So können die antimikrobiellen und antientzündlichen Eigenschaften der mineralischen Verbindung durch Einarbeitung ausgewählter Substanzen mit antientzündlichen und/oder antioxidativen Eigenschaften wie z.B. aus der Klasse der Phytochemcials erhöht und gegebenenfalls entstehende freie Radikale reduziert werden.

Das vorliegende Wechsellagerungstonmineral ermöglicht somit eine Mehrfach-Wirkstrategie und weist gegenüber der aktuellen Medikation den Vorteil auf, dass potentielle Arzneimittelnebenwirkungen erheblich reduziert werden können.

Das vorliegend verwendete Wechsellagerungstonmineral weist aufgrund ihrer einzigartigen mineralogischen Zusammensetzung besondere Eigenschaften auf, die für die Gesunderhaltung und Therapie von Mensch und Tier interessant sind. Zu den besonderen Eigenschaften gehören die physikalische Absorption von bioaktiven Stoffen, die chemische Absorption von Schadstoffen, die Bildung von Gelen aufgrund der Quellfähigkeit des enthaltenen Tonminerals, die Thixotropie und die Kationen- und Anionen-Austauschfähigkeit.

Das bevorzugt verwendete Wechsellagerungstonmineral ist in der Lage, Durchfall bei Kälbern und anderen Tieren nach kurzer Zeit ohne jegliche Nebenwirkung zu stoppen. Die kanzerogen wirksamen Mykotoxine, wie Aflatoxin B1 und Okratoxin werden zu 99% bzw. 85% fest gebunden. Ebenso werden Stoffwechselgifte, wie Ammonium im Magen-DarmTrakt durch Kationenaustausch an der mineralischen Verbindung adsorbiert und mit dieser ausgeschieden. Mittels physikalischer Absorption ist es möglich, bioaktive Stoffe aus Pflanzen an die mineralische Verbindung zu binden, die anschließend im Darm unter den dort herrschenden Bedingungen freigesetzt werden können. In einem Rattenmodell mit einer künstlich erzeugten Entzündung konnten durch Zugabe einer Suspension aus der mineralischen Verbindung die Entzündungsparameter vergleichbar mit der aktuellen Medikation deutlich reduziert werden. So konnte in Versuchen mit einem Ratten-Kurzzeitschädigungsmodell überraschend festgestellt werden, dass die verwendete mineralische Verbindung entzündungshemmend wirkt und es gleichzeitig zu einer Mukosaheilung kommt, wie es eine histologische Begutachtung gezeigt hat.

Mineralien kann man in 10 unterschiedliche Klassen einteilen, wobei jede Klasse für sich wieder in mehreren verschiedenen Unterklassen aufgeteilt werden kann. Viele Mineralien haben ihre bevorzugten Einsatzgebiete. Für adsorptive Prozesse kommen insbesondere aus der Silikatgruppe das Gerüstsilikat Zeolith und das Schichtsilikat Montmorillonit zum Einsatz. Zur besseren Vergleichbarkeit der verschiedenen Montmorillonitqualitäten wurde der Begriff Bentonit geprägt, dessen mineralogische Zusammensetzung mindestens 60 bis 70% Montmorillonit enthalten muss. Für viele technische Anwendungen ist diese Gruppenbildung sehr hilfreich. Die Wirkung von Mineralen in biologischen Systemen geht aber weit über Adsorptionsprozessen hinaus. Deshalb ist die alleinige Fixierung auf nur einen möglichst hohen Montmorillonitanteil in der mineralischen Verbindung nicht zielführend.

Die häufig in der Natur vorkommenden mineralischen Verbindungen bzw. Matrizen, mit Wechsellagerungstonminerale (Mixedlayer) als Hauptbestandteil, werden auf Grund ihrer im Vergleich zu reinen Montmorillonitschichten geringeren messbaren spezifischen Oberflächen, Quellvermögen und Kationenaustauschkapazitäten seltener für adsorptive und katalytische Applikationen verwandt.

Es hat sich nunmehr herausgestellt, dass natürlich vorkommende mineralische Verbindungen bzw. Matrizen mariner Genese aus Mixedlayer oder Wechsellagerungsmineralien bestehen, die sich aus quellfähigen und nichtquellfähigen Schichten in unregelmäßiger Folge zusammensetzen und die ggf. noch anderen Minerale wie Silikate, Oxide, Carbonate, Sulfide und Sulfate enthalten, nach einer entsprechenden Aufbereitung durchaus adsorptive und andere interessante Eigenschaften aufweisen.

Mixedlayer können strukturell aus sehr unterschiedlichen Wechsellagerungsschichten bestehen z.B. Kaolinit/Smectit, Chlorit/Vermikulit, Glimmer/Vermikulit oder sehr häufig Wechsellagerung von Illit/Smectit oder Illit/Montmorillonit. Dadurch sind vielfältigere Austauschreaktionen von Kationen und Anionen möglich als bei reinen Montmorilloniten. Deshalb sind Mixedlayer in einer Kombination mit anderen reaktiven Mineralen besonders gut geeignet für die Bindung von in Lösung befindlichen Stoffen in biologischen Systemen.

Erfindungsgemäß wird daher eine mineralische Verbindung bzw. ein Mineral verwendet, die bzw. das mindestens ein Wechsellagerungstonmineral umfasst.

Besonders bevorzugt kommt dabei ein Wechsellagerungstonmineral aus Montmorillonit und Illit/Muskovit zum Einsatz, wobei in diesem Wechsellagerungsmineral Montmorillonit und Illit/Muskovit in einem Verhältnis von 60:40 bis 40:60 enthalten sein können, wobei ein Verhältnis von 50:50 bevorzugt ist. Dass heißt, das Montmorillonit und Illit/Muskovit zu jeweils 50 Gew% enthalten sein können.

Zusätzlich zu den Mineralien Montmorillonit und Illit/Muskovit kann die bevorzugt verwendete mineralische Verbindung auch Anteile von anderen Tonmineralien, wie Kaolinite und Chlorite, Carbonate, Sulfide, Oxide und Sulfate aufweisen.
In einer besonders bevorzugten Ausführungsform umfasst das vorliegende Wechsellagerungstonmineral durchschnittlich 50-60 Gew%, bevorzugt 55 Gew% Montmorillonit-Muskovit-Wechsellagerung, 15-25 Gew%, bevorzugt 20 Gew % Illit, 5-9 Gew%, bevorzugt 5 Gew% Kaolinit/Chlorit, 10-20 Gew%, bevorzugt 15 Gew% Quarz, 1-2 Gew%, bevorzugt 1 Gew% Calcit, 0,9-1,5, bevorzugt 1 Gew% Dolomit, 0,9-1,9 Gew%, bevorzugt 1 Gew% Feldspat, 0,9 - 1,0 Gew%, bevorzugt 1 Gew% Pyrit und 0,6-1,0 Gew%, bevorzugt 1 Gew% Gips.

Die chemische Zusammensetzung der Hauptelemente kann in Gew% wie folgt angegeben werden: SiO₂ 58,9; TiO₂ 0,9; Al₂O₃ 17,9; Fe₂O₃ 6,3; MgO 2,0; CaO 0,3; Na₂O 1,1; K₂O 3,0; P₂O₅ 0,1; Sonstige 9,5.

Ein Teil der in der mineralischen Verbindung enthaltenen Minerale geht bei Vorhandensein eines Lösungsmittels in dieses über. So ergab ein Suspensionsversuch mit 2 Gew% mineralischer Verbindung in destilliertem Wasser folgende Ergebnisse: Leitfähigkeit 346 µS; pH-Wert 7,3; Kalium 5 mg/l; Natrium 73 mg/l; Chlor 20 mg/l; Magnesium 0 mg/l; Kalzium 1 mg/l; SO₄ 121 mg/l; Aluminium 0 mg/l; SiO₂ 8 mg/l; Eisen 0 mg/l.

Besonders hervorzuheben ist der hohe Anteil an Sulfaten und die Tatsache, dass keine Aluminium-Ionen in Lösung gehen. Die wässrige Tonmineral-Suspension ist demnach frei von Aluminium-Ionen. Dies ist besonders für die humane Anwendung von besonderer Bedeutung.

Eine weitere Besonderheit der bevorzugten Ausführungsform ist der hohe Anteil an organischem Kohlenstoff von 0,4 Gew%. Zusammen mit den vorhandenen Sulfaten und Pyriten ist dies ein wichtiger Beweis für die marine Genese der mineralischen Verbindung und gleichzeitig ein wesentlicher Beitrag zur Wirksamkeit derselben.

Die bevorzugt verwendete mineralische Verbindung weist eine innere (BET)-Oberfläche von 50 -100 m²/g, bevorzugt 55 - 65 m²/g, insbesondere bevorzugt von 60 m²/g auf. Die innere Oberfläche der bevorzugt verwendeten mineralischen Verbindung ist somit z.B. im Vergleich zu den hochquellfähigen Montmorilloniten relativ gering. Die mittlere Teilchengröße der bevorzugt verwendeten mineralischen Verbindung kann in einem Bereich von 1 bis 10 µm, insbesondere bei 2 µm liegen. Nach thermischer Behandlung des Tonminerals kann die innere BET-Oberfläche auf 100 bis 200 m²/g ansteigen.

Das bevorzugt verwendete Wechsellagerungstonmineral wird aus den in Deutschland in Mecklenburg-Vorpommern, genauer in der Nähe von Friedland im östlichen Teil der Mecklenburgischen Seenplatte, vorhandenen Tonvorkommen isoliert und aufbereitet.

Das bevorzugt verwendete Wechsellagerungstonmineral weist, wie bereits oben erwähnt, einzigartige Eigenschaften auf, in denen es sich von anderen mineralischen Matrizen wie Bentonit oder Montmorillonit wesentlich unterscheidet.

So ist das vorliegend verwendete Wechsellagerungstonmineral nicht nur in der Lage, Kationen in den vorhandenen Montmorillonitschichten auszutauschen, sondern auch Anionen zu binden. Der Wirkmechanismus der vorliegend zum Einsatz kommenden mineralischen Verbindung ist daher ein anderer als bei Bentoniten. So werden z.B. anionische Stoffe besonders gut an den Bruchkanten der in der verwendeten mineralischen Verbindung enthaltenen Illite/Muskovite locker gebunden. Ursache dafür sind fehlende bzw. herausgeschlagene Kaliumionen, die im Mineralverbund für einen Ladungsausgleich sorgen. So entstehen positive Ladungen, an denen Anionen locker gebunden werden können. Da bioaktive Anionen eine relativ hohe Molekularmasse und damit Durchmesser aufweisen, sind die an den Bruchkanten entstandenen Vertiefungen nicht groß genug, um eine feste Bindung zu realisieren. Dies gelingt nur mit kleineren Molekülen, wie die sehr hohe Adsorptionskraft für spezielle Anionen beweist.

Um eine möglichst hohe Bindungskapazität des vorliegenden Wechsellagerungstonmineral zu erreichen, ist eine Feinstzerkleinerung zur Schaffung einer möglichst hohen Anzahl von Bruchkanten vorteilhaft. Die bevorzugte Teilchengröße liegt dabei bei ca. 2 µm. Zudem hat sich im Rahmen von Zetapotentialuntersuchungen herausgestellt, dass durch die Feinstzerkleinerung z.B. mittels einer Gegenstrahlmühle sich die Summenladung bei pH 7 von -140 auf -20mV verändert, was auf eine starke Zunahme der positiven Andockstellen für Anionen hindeutet.

Bevorzugterweise wird dem mindestens einem Wechsellagerungstonmineral mindestens ein mehrwertiges Kation, insbesondere einen zwei- oder dreiwertigem Kation, zugegeben bzw. wird eine Mischung aus Tonmineral und Kation hergestellt. Das mehrwertige Kation ist dabei bevorzugterweise ausgewählt aus einer Gruppe enthaltend Fe²⁺, Ca²⁺, Mg²⁺, Fe³⁺ und Al³⁺. Durch Zugabe der mehrwertigen Kationen wird insbesondere die Bindungsaffinität des Tonminerals gegenüber den biologischen oder chemischen Darmtoxinen erhöht.

Das mehrwertige Kation kann in Form eines Chlorid-Salzes und/oder Sulfat-Salzes zugegeben, wobei besonders die Zugabe von M₉Cl₂ und MgSO₄ bevorzugt ist.

Von Bedeutung für die Anwendbarkeit des vorliegenden Wechsellagerungstonminerals bei der Behandlung von CED sind deren spezifischen Quelleigenschaften. So weist die vorliegende mineralische Verbindung bis zu einem pH-Wert von 5 schlechte und erst ab einem pH-Wert über 6,5 gute Quelleigenschaften aufweist. Dies bedeutet, dass die an der mineralischen Verbindung ggf. gebundenen weiteren Substanzen wie z.B. Phytopharmaka und/oder Antioxidantien im Magen in der mineralischen Verbindung geschützt bleiben und erst im Darm bei höherem pH-Wert wieder freigesetzt werden. Die vorliegende mineralische Verbindung kann daher auch als ein natürliches Drug Delivery System beschrieben werden.

Auch wird das Wechsellagerungstonmineral in bevorzugter Weise in Form einer sterilisierten Mineralsuspension verwendet, die z.B. mittels Autoklavierung sterilisierbar ist. Das Wechsellagerungstonmineral kann auch in einer kolloid dispersen und/oder gelartigen Form verwendet werden.

In einer Ausführungsformist die mindestens eine weitere Substanz, mit der die mineralische Verbindung modifiziert bzw. versehen ist, eine organische oder anorganische Substanz, wobei die mindestens eine weitere Substanz auch eine entzündungshemmende Substanz sein kann.

Bevorzugterweise ist die mindestens eine weitere Substanz ausgewählt aus einer Gruppe enthaltend Pflanzenextrakte, Fermente, Harze, Antioxidantien, Aktivkohle, Huminsäuren und deren Salze.

Das mindestens eine Pflanzenextrakt, bevorzugt ein Pflanzenextrakt aus der Gruppe der Phytopharmaka kann ausgewählt sein aus einer Gruppe enthaltend Grünalgen, Obst, Gemüse, Kräuter, Heilkräuter, Gewürze und Pilze.

In einer weiteren Ausführungsform ist das mindestens eine Pflanzenextrakt ein polyphenolreiches Pflanzenextrakt. Insbesondere polyphenolreiche Pflanzenextrakte sind sowohl für ihre antibakterielle als auch antivirale Wirksamkeit bekannt und können somit bakterielle und virale Infektionserreger, die bevorzugt durch die Mundhöhle und damit auch in den Verdauungstrakt eines menschlichen Körper gelangen, bekämpfen und Erkrankungen vorbeugen.

Als geeignete Pflanzenextrakte können Extrakte aus der Pflanze der Gattung der Zistrosen wie z.B *Cistus incanus (Cistus creticus), Cistus landanifer L., Cistus monospeliensis L., Cistus laurifolius L., Cistus parviflorus L., Cistus salviifolius L.* oder aus der Gattung der Geranien (Storchschnäbel) *wie z.B. Geranium sanguineum L., Geranium canariense, Geranium columbinum L, Geranium endressii, Geranium himalayense, Geranium sibiricum, Geranium pyrenaicum Burm., Geranium thunbergii* verwendet werden.

Besonders bevorzugt sind auch Extrakte aus Pflanzen der Gattung der Echinacea (Sonnenhüte), insbesondere *Echinacea angustifolia, Echinacea atrorubens, Echinacea laevigata, Echinacea pallida, Echinacea paradoxa, Echinacea purpurea, Echinacea sanguinea, Echinacea simulata* und/oder *Echinacea tennesseensis.*

Weitere geeignete Pflanzen sind Salbei (Salvia) wie z.B. *Salvia aegyptiaca L., Salvia africana L., Salvia algeriensis, Salvia apian Jeps., Salvia aurea L., Salvia buchananii, Salvia cacaliaefolia, Salvia canariensis L., Salvia chiapensis, Salvia discolor, Salvia lanceolata, Salvia officinalis, Salvia sinaloensis* oder *Salvia splendens.*

Es ist ebenfalls möglich Eucalyptus, insbesondere *Eucalyptus globulus, Eucalyptus citriodora, Eucalyptus radiata,* bevorzugt in Form eines Öls zu verwenden.

Weitere geeignete Pflanzenextrakte können Granatäpfel z.B. *Punica granatum, Punica protopunica;* Hamamelis, z.B. *Hamamelis virginiana, Hamamelis vernalis, Hamamelis ovalis, Hamamelis japonica, Hamamelis mollis;* Zitronenmelisse z.B. *Melissa officinalis;* Kamille, z.B. *Matricaria chamomilla L.*; Ginseng, z.B. *Panax ginseng;* Minze z.B. *Mentha aquatica L., Mentha austriaca, Mentha australis, Mentha japonica, Mentha longifolia L., Mentha requienii, Mentha cordifolia, Mentha suaveolens*; Rosmarin z.B. *Rosmarinus officinalis* L.; Rhababerwurzel wie Rhabarberwurzelextrakt; Gelbwurzel (*Curcumo longa*)*,* und/ oder Tee z.B. Grüner Tee sein.

In einer weiteren Ausführungsform des Wechsellagerungstonminerals können Antioxidantien zugesetzt werden, die die Fähigkeit besitzen, die sich in lebenden Organismus aufgrund von oxidativen Prozessen bildenden hochreaktive freie Radikale zu deaktivieren. Diese Radikale beeinflussen und beschleunigen den Alterungsprozess von Organismen und beeinträchtigen die Gesundheit. So sind vermutlich Radikale die Ursache für Arteriosklerose und Krebs.

In der biologischen Forschung, Medizin, Pharmazie und Lebensmitteltechnologie wird dabei zunehmend das antioxidative Potential von natürlich vorkommenden Antioxidantien untersucht. Eine reichhaltige Quelle an antioxidativ wirksamen Substanzen stellen dabei Pflanzen dar, insbesondere Gemüsepflanzen und medizinische Kräuter.

Typische in Pflanzenextrakten vorkommene Verbindungen mit hohem antioxidativen Potential sind Polyphenole und deren Derivate, insbesondere Flavonoide z.B. Dimethoxyflavone, Naphthoflavone, Quercetin, 3-O-Methylquercetin, Myricetin, Catechine, Oligomere der Catechine; Cumarine z.B. Umbelliferon, Herniarin, Aesculetin, Cumarin, Scopoletin, Isoscopoletin; Terpene z.B. dicyclische Terpene wie Carnosol, Carnosolsäure; Labdanolsäure, Labdandiol, Ribenol, Laurifolinsäure; Sesquiterpene, Sesquiterpenlactone, Seqsquiterpenalkohole z.B. Germacren, Copaen, Bisabolol, Farnesen, Matricarin, Matricin Tetraterpene wie z.B. Carotonoide wie Lycopin, Betacarotin, Lutein oder einfache Terpene wie z.B. Cineol, Geranial, Citronellal, Nerol, Linalool.

Auch weisen verschiedene Vitamine ein hohes antioxidatives Potential auf. Hervorzuheben sind hierbei insbesondere Vitamin C und Vitamin E.

Die Pflanzenextrakte und/ oder Antioxidantien werden bevorzugt in einer Menge zwischen 20 und 50 Gew% bezogen auf das Gesamtgewicht der mineralischen Verbindung verwendet.

Wie oben erwähnt kann die mindestens eine weitere Substanz zur Modifizierung der mineralischen Verbindung aus der Gruppe der Huminsäuren ausgewählt sein. In einer Ausführungsform umfassen die vorliegend zum Einsatz kommenden Huminsäuren wasserlösliche Fulvosäuren z.B. mit einer Molmasse kleiner 3000 Da, wasserunlösliche, aber alkalilösliche Huminsäuren und wasser- und alkaliunlösliche Huminstoffe. Generell können die verwendeten Huminsäuren Molmassen zwischen 1000 und 200.000 Da aufweisen.

Huminsäuren sind dreidimensionale Makromoleküle mit heterogen verknüpften Bausteinen. Huminsäuren weisen im Allgemeinen zwei charakteristische Bereiche oder Abschnitte auf: ein zentraler Kern mit hohem Aromatizitätsgrad sowie starken Vernetzungen und periphere, durch Brückenbindungen verknüpfte funktionelle Gruppen. So liegen polyionische Strukturen mit z. B. Carbonsäureester-, phenolischen Hydroxyl-, Carbonyl- und Carboxyl-Gruppen vor. Auch Amino- und Sulfhydrylreste sowie chinoide und flavonoide Strukturen sind vorhanden. Besonders die aus pflanzlichen Produkten entstandenen Huminsäuren, so auch BraunkohleHuminsäuren, weisen zusätzlich Flavonstrukturen auf.

Dies begründet bestimmte pharmakologische Wirkungen (antiphlogistisch, zellabdichtend, viruzid, fungizid). Je nach physikalischer Beschaffenheit der verschiedenen Huminsäureprodukte variieren ihre Adsorptionseigenschaften und lonenaustauscheffekte gegenüber physiologisch aktiven Substanzen.

Huminsäuren bilden den Hauptanteil der Huminstoffe, denen wir überall in der Natur begegnen: in Böden, im Torf, in der Braunkohle und in den Pflanzen selbst. Sie sind auch in vielen Lebensmitteln enthalten. Der Ton-Humus-Komplex im Boden ist eine wichtige Voraussetzung für ein gesundes Pflanzenwachstum. Huminsäuren sind der natürliche Synergiepartner für die mineralische Verbindung, da sie durch zusätzliche Wirkmechanismen Einfluss auf die Therapie von Erkrankungen der Verdauungsorgane und von Störungen des Verdauungsstoffwechsels nehmen. Durch ihre adstringierende, antiphlogistische, antiresorptiven, antibakteriellen und viruziden Wirkungen ergänzen die Huminsäuren das Wirkungsspektrum der mineralischen Matrix in optimaler Weise.

Die erfindungsgemäß verwendeten Huminsäuren können z.B. aus einer Braunkohlelagerstätte gewonnen werden. Beispielhaft sei hier auf den Typ Weinböhler Huminsäure 67 (WH67) hingewiesen, der aus spezifischen Braunkohlelagerstätten gewonnen wird. Diese Braunkohlehuminsäuren weisen wie alle aus pflanzlichen Produkten entstandenen Huminsäuren besondere Flavonstrukturen, u.a. Fisetin, Quercetin, Flavone und Xanthine, auf. Sie sind in verschiedenen Ausführungsformen verfügbar. So kann die Verteilung der Molmassen der Huminsäuren zu 27% zwischen 10 und 50kD, zu 32% zwischen 50 und 100kD und zu 36% bei bis 150kD. Der Rest von 5% reicht bis zu 200kD. Niedermolekulare Verbindungen sind bevorzugterweise nicht enthalten.

Wie auch oben erwähnt kann die mindestens eine weitere Substanz zur Modifizierung der mineralischen Verbindung eine anorganische Substanz sein, die bevorzugterweise ausgewählt ist aus der Gruppe der Zeolithe, der hochquellfähigen Tonminerale und der Metallsalze, insbesondere der Calcium-, Natrium- und Magnesiumsalze.

Als Metallsalzkomplexbildner kommen insbesondere solche Stoffe zum Einsatz, die den Elektrolythaushalt des Menschen sinnvoll ergänzen. So konnte bei Untersuchungen festgestellt werden, dass sich die Permeabilität der Darmschleimhaut bei Magnesiummangel stark erhöht. Deshalb wird im Rahmen der CED bevorzugt Magnesiumchlorid verwendet.

In einer weiteren bevorzugten Ausführungsform wird die mineralische Verbindung unter Verwendung der Metallsalze bzw. Metallsalzkomplexbildnern und den Huminsäuren zu einem Bio-Mineralischen Wirkkomplex verbunden.

Diese Bio-Mineralischen Wirkkomplexe können je nach vorgesehener Anwendung eine mineralische Verbindung von 20 bis 40 Ma%, Huminsäure von 25 bis 40 Ma% und Metallsalz 20 bis 30 Ma% enthalten.

Auch wird die vorliegende mineralische Verbindung bei Verwendung in einem therapeutischen Verfahren, insbesondere bei Verwendung zur Vorbeugung und Behandlung von CED mindestens in einer Einmaldosis mit einer Menge zwischen 1 bis 10 g, bevorzugt 2 bis 8 g, insbesondere bevorzugt 3 bis 5 g bis zu 3x täglich verabreicht.

Die vorliegende Erfindung wird anhand der folgenden Ausführungsbeispiele unter Bezugnahme auf die Figuren näher erläutert. Es zeigen:
- Fig. 1: ein erstes Diagramm zum Nachweis der Wirkung des vorliegend verwendeten Tonminerals in einem Ratten-Kurzeitschädigungsmodell;
- Fig. 2: ein zweites Diagramm zum Nachweis der Wirkung des vorliegend verwendeten Tonminerals in einem Ratten-Kurzeitschädigungsmodell; und
- Fig. 3: ein drittes Diagramm zum Nachweis der Wirkung des vorliegend verwendeten Tonminerals in einem Ratten-Kurzeitschädigungsmodell; und
- Fig. 4: ein viertes Diagramm zum Nachweis der Wirkung des vorliegend verwendeten Tonminerals in einem Ratten-Kurzeitschädigungsmodell;
- Fig. 5: ein fünftes Diagramm zum Nachweis der Wirkung des vorliegend verwendeten Tonminerals in einem Ratten-Kurzeitschädigungsmodell, und
- Fig. 6A-C: weitere Diagramme zum Nachweis der Wirkung des vorliegend verwendeten unbehandelten Tonminerals und einer thermisch behandelten Variante des Tonminerals.

### Ausführungsbeispiel 1a: Stoffparameter des verwendeten Wechsellagerungstonminerals (bezeichnet als Resormin)

Korngrößenmessung mit CILAS 1064 nass, Messbereich: 0,04 µm - 500,00 µm / 100 Klassen, Flüssigkeit: Wasser + Na-Pyrophosphat, Messmethode: Laserbeugung

Die Korngrößenverteilung beträgt < 0,50 µm 2,4 Masse%, < 1 µm 18 Masse%, < 2 µm 67 Masse%, < 3 µm 91 Masse%, < 4 µm 99 Masse%, < 6 µm 100 Masse%.

Die verwendete mineralische Verbindung hat ein Gesamtanteil an Tonmineralien von 80% und enthält durchschnittlich 55 Gew% Montmorillonit-Muskovit-Wechsellagerung im Verhältnis 60:40, 20 Gew % Illit, 5 Gew% Kaolinit/Chlorit, 15 Gew% Quarz, 1 Gew% Calcit, 1 Gew% Dolomit, 1 Gew% Feldspat, 1 Gew% Pyrit und 1 Gew% Gips.

Die chemische Zusammensetzung der Hauptelemente kann in Gew% wie folgt angegeben werden: SiO₂ 58,9%; TiO₂ 0,9%; Al₂O₃ 17,9%; Fe₂O₃ 6,3%; MgO 2,0%; CaO 0,3%; Na₂O 1,1%; K₂O 3,0%; P₂O₅ 0,1%; Sonstige 9,5%.

Die Schüttdichte beträgt 200 g/l, die spezifische Oberfläche 65 m²/g, der pH-Wert 8,3, die Feuchtigkeit 5%, die H₂O-Absorption 150% Enslin nach 60 min, Zetapotential mittels DT-300-Messung bei pH 7 -20mV, der Gehalt an Dioxine bei 0,2 ng/kg, und KationenAustauschkapazität 85 mval/100g.

### Ausführungsbeispiel 1b: Stoffparameter des thermisch behandelten Wechsellagerungstonminerals

Das im Ausführungsbeispiel 1a beschriebene Wechsellagerungstonmineral wurde für 1 h bei 350°C thermisch behandelt (calciniert). Das so modifizierte Tonmineral wird im Folgenden als Fl5pp bezeichnet.

### Ausführungsbeispiel 2: Ratten-Kurzzeitschädigungs-Modell

Zum Nachweis der Wirksamkeit der mineralischen Verbindung wurde das therapeutische und prophylaktische Potential in einem in vivo Ratten-Kurzzeitschädigungs-Modell untersucht. Dazu wurde in einem definierten Dünndarmsegment mit intaktem vaskulärem System durch Verabreichung von lodoacetamid (1%,15 min) eine Entzündung induziert. Anschließend erfolgt die Perfusion des geschädigten Darmsegments mit einem Kontrollmedium (z.B. Dulbeccos Modified Eagle Medium, DMEM) oder einer therapeutischen Zusammensetzung wie Mutaflor, Dexamethason und der vorliegenden mineralischen Verbindung für zwei Stunden.

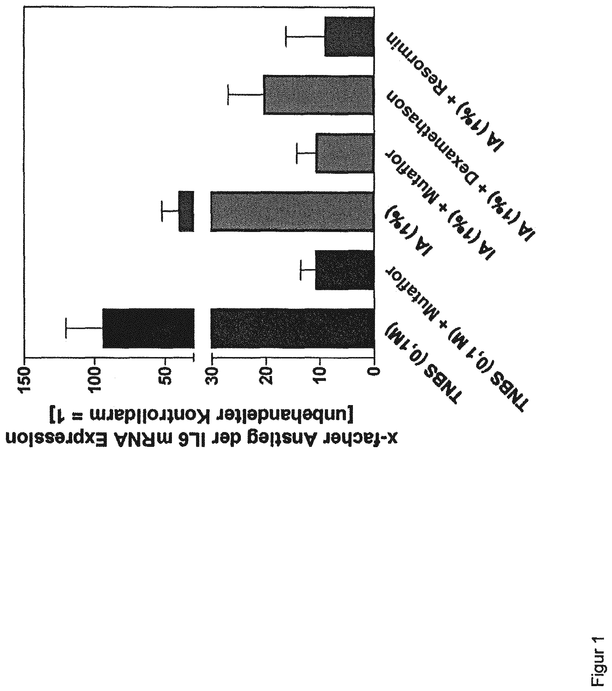

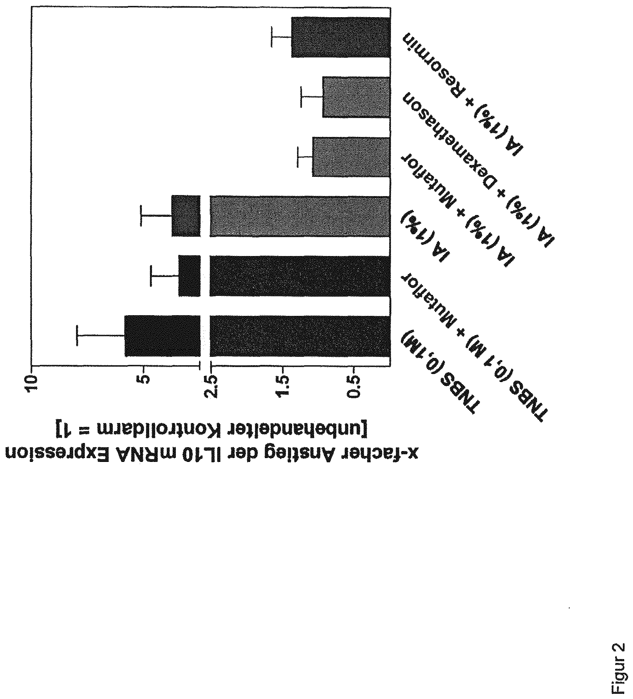

Die im Darm der Ratte induzierte Entzündung wird u.a. durch die mRNA Expression der Interleukine IL-6 (Figur 1) und IL-10 (Figur 2) nachgewiesen, die bei Vorliegen einer Entzündung stark ansteigt. Dieser Anstieg der Expression wird jedoch durch Zugabe der therapeutischen Zusammensetzungen deutlich reduziert.

Aus dem Diagramm der Figur 1 ist entnehmbar, dass die bei Verabreichung von lodoacetamid (IA) die IL6 mRNA Expression ansteigt, jedoch bei gleichzeitiger Gabe der bekannten Medikamente Mutaflor und Dexamethason der Anstieg der IL6 mRNA Expression stark reduziert ist. Auch bei gleichzeitiger Verabreichung von IA und der vorliegenden mineralischen Verbindung Resormin wird die IL6 mRNA Expression auf ca. 20% des Expressionswertes reduziert. Die Wirksamkeit der vorliegenden mineralischen Verbindung Resormin bei der Reduktion der IL6 mRNA Expression übersteigt zudem die Wirksamkeit von Dexamethason und Mutaflor.

Ein vergleichbares Bild ist dem Diagramm der Figur 2 zu entnehmen, die den Einfluss der getesteten Therapeutika auf die IL 10 mRNA Expression zeigen. Auch hier ist eine mit dem bekannten Medikamentes Mutaflor und Dexamethason vergleichbare Wirkung der vorliegenden mineralischen Verbindung Resormin nachweisbar.

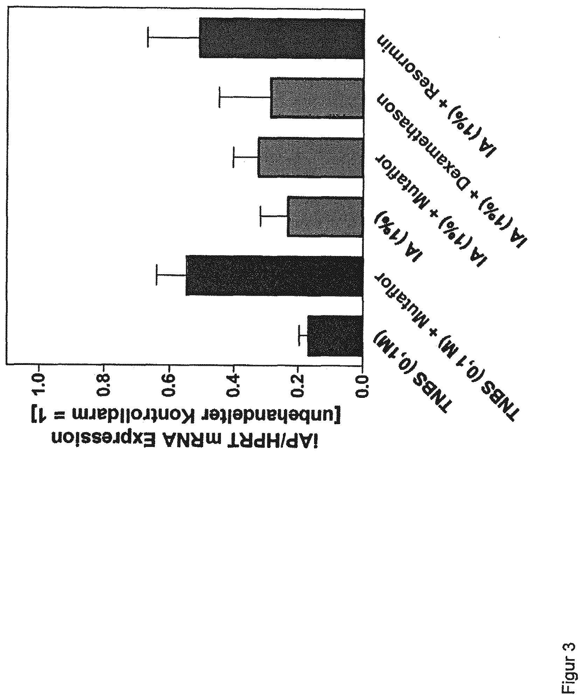

Das Diagramm der Figur 3 wiederum zeigt den Einfluss der untersuchten Zusammensetzungen auf die mRNA Expression von iAP (intestinale Alkaline Phosphatase) als Indikator für die Darmregeneration. Hier bewirkt die gleichzeitige Verabreichung von IA und den therapeutisch wirksamen Zusammensetzungen Mutaflor und Dexamethason einen leichten Anstieg des iAP mRNA Expression während die gleichzeitige Verabreichung von IA und der mineralischen Verbindung Resormin eine im Vergleich zu den bekannten Medikamenten eine um das ca. 2fach erhöhte iAP mRNA Expression bewirkt. Somit zeigt Resormin hier sogar eine gegenüber den bekannten Mitteln wesentlich verbesserte Wirksamkeit.

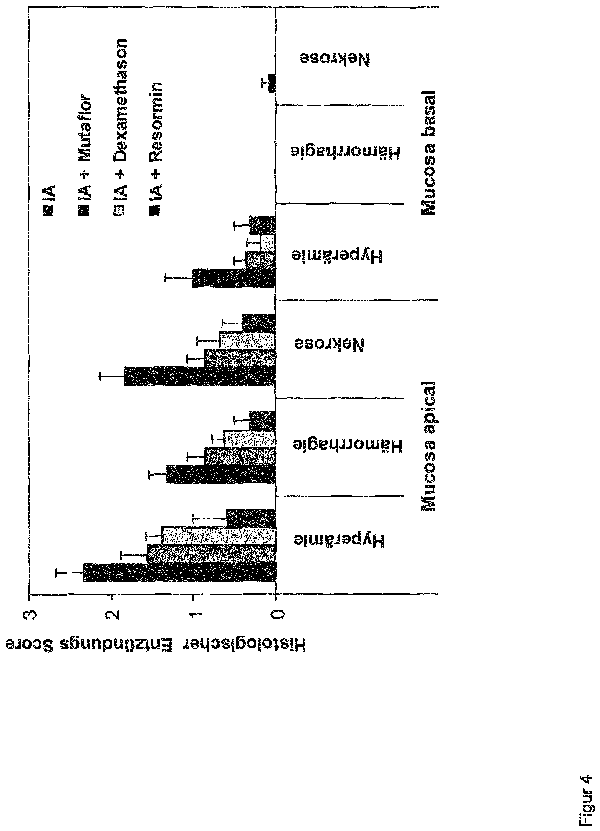

Das Diagramm der Figur 4 zeigt das Ergebnis der Bewertung der einzelnen histologischen Parameter bei der Ermittlung eines Histo-Scores von Schnitten des beanspruchten Dünndarmsegmentes. Dazu wurde die Mucosa apical und basal hinsichtlich der Hemorrhage (Einblutungen), der Hyperemia (gefüllte Blutgefäße) und der Necrosis (totes Gewebe) auf einer Punktescala von 0 bis 3 bewertet. In fast allen bewerteten Punkten konnte Resormin im Vergleich zu Dexamethason und Mutaflor wesentlich besser abschneiden.

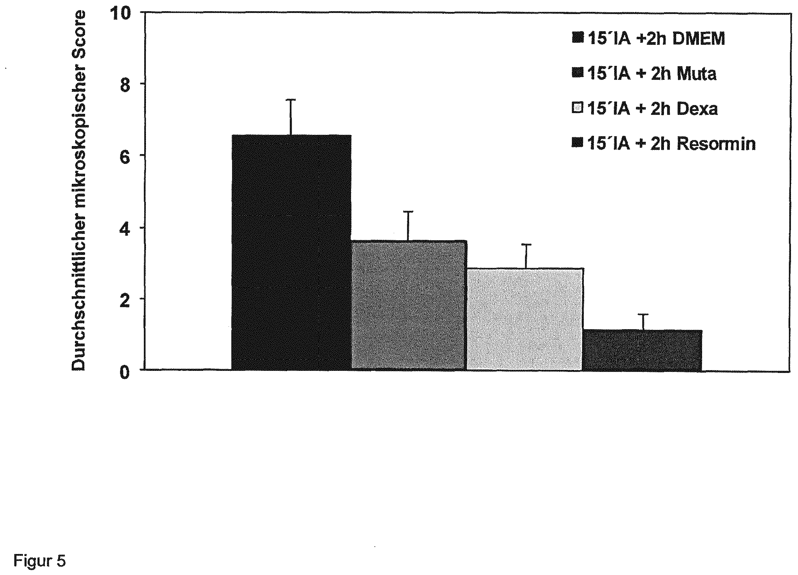

Das Diagramm der Figur 5 zeigt den Gesamt-Histo-Score für die bewerteten Parameter. Hier zeigt sich sehr deutlich, dass Resormin gegenüber den Vergleichsprodukten wesentlich besser abschneidet. Bei einem Score von 1 kann man schon von einer Mucosaheilung sprechen. Demzufolge war die mineralische Verbindung Resormin in der Lage, innerhalb von 2 Stunden die Mocosa wieder vollständig herzustellen.

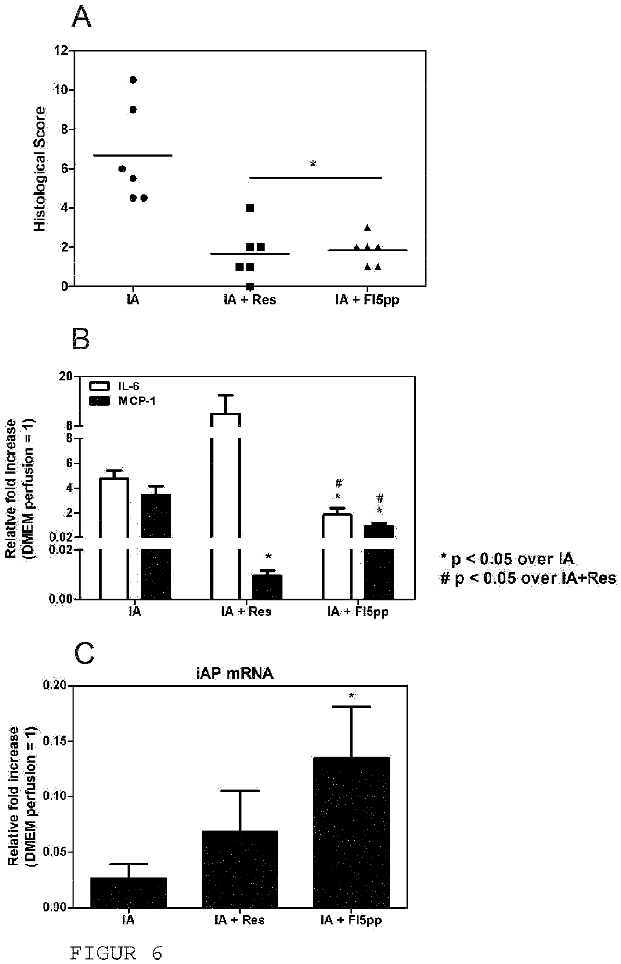

Die in den Figuren 6A bis 6C gezeigten Diagramme belegen die Wirksamkeit des unbehandelten Tonminerals Resormin (Res) als auch die Wirksamkeit des thermisch behandelten Tonminerals Fl5pp, das wie in Ausführungsbeispiel 1b beschrieben gewonnen wurde.

Das Diagramm der Figur 6A zeigt den histologischen Score in Gegenwart von lodoacetamid (IA) als die Darmwand schädigendes Agens allein und in Kombination mit unbehandelten Resormin und thermisch behandelten Tonmineral FI5pp. Sowohl Resormin als auch FI5pp zeigen einen deutlich erniedrigten histologischen Score gegenüber dem mit IA geschädigten Modell, unterscheiden sich in ihrer Wirksamkeit bezüglich des histologischen Scores jedoch nicht voneinander (Fig. 6A).

Hinsichtlich der Wirkung von Resormin und Fl5pp auf die IL6 mRNA Expression ist eine deutliche Reduzierung der IL6 mRNA Expression durch beide Tonmineralien nachweisbar, wobei Resormin eine stärkere Wirkung aufweist als Fl5pp (Fig. 6B).

Aus Fig. 6C ist wiederum die Wirkung von Resormin und Fl5pp auf die mRNA Expression der intestinalen alkalischen Phosphatase (iAP) gezeigt. Die mRNA Expression von iAP gilt als ein Indikator für die Darmregeneration und die Genesung der Darmwand. Die gleichzeitige Verabreichung von IA+ Resormin und IA+FI5pp führt zu einem deutlichen Anstieg der iAP- mRNA Expression. Das thermisch behandelte Fl5pp zeigt hier im Vergleich zu dem thermisch unbehandelten Resormin einen deutlichen Anstieg der iAP-mRNA Expression, was auf eine verbesserte Wirkung des thermisch behandelten Tonminerals bei der Darmgeneration hinweist.

## Patentansprüche

1. Wechsellagerungstonmineral zur Verwendung als Mittel zur Prävention und *l* oder Behandlung von chronisch-entzündlichen Darmerkrankungen (CEO), **dadurch gekennzeichnet, dass** das Tonmineral vor der Verwendung auf eine Partikelgröße kleiner 5 µm gemahlen wird und anschließend thermisch bei einer Temperatur zwischen 300°C und 500°C, bevorzugt zwischen 350°C und 450°C, über einen Zeitraum von 1h bis 5h, bevorzugt 1 h bis 3h behandelt wird.

2. Wechsellagerungstonmineral zur Verwendung nach Anspruch 1, **dadurch gekennzeichnet, dass** das Wechsellagerungstonmineral, ein Wechsellagerungstonmineral aus Montmorillonit und Illit/Muskovit umfasst.

3. Wechsellagerungstonmineral zur Verwendung nach Anspruch 1 oder 2, **dadurch gekennzeichnet, dass** das Wechsellagerungstonmineral die Tonminerale Montmorillonit und Illit/Muskovit zu jeweils mindestens 50 Gew% umfasst.

4. Wechsellagerungstonmineral zur Verwendung nach einem der vorhergehenden Ansprüche, **dadurch gekennzeichnet, dass** weitere Tonminerale wie Kaolinite und Chlorite enthalten sind.

5. Wechsellagerungstonmineral zur Verwendung nach einem der vorhergehenden Ansprüche, **dadurch gekennzeichnet, dass** die mineralische Verbindung 50-60 Gew% Montmorillonit-Muskovit-WL, 15-25 Gew% Illit/Muskovit, 5-9 Gew% Kaolinit/Chlorit, 10-20 Gew% Quarz, 1-2 Gew% Calcit, 0,9-1,5 Gew% Dolomit, 0,9-1,9 Gew% Feldspat, 0,9-2,0 Gew% Pyrit und 0,6-1,0 Gew% Gips umfasst.

6. Wechsellagerungstonmineral zur Verwendung nach einem der vorhergehenden Ansprüche, **dadurch gekennzeichnet, dass** die Matrix eine BET-Oberfläche von 50-100 m²/g, bevorzugt 55-65 m²/g, insbesondere bevorzugt von 60 m²/g aufweist.

7. Wechsellagerungstonmineral zur Verwendung nach einem der vorhergehenden Ansprüche, **dadurch gekennzeichnet, dass** die mineralische Verbindung in einer kolloid dispersen und/oder gelartigen Form verwendet wird.

8. Wechsellagerungstonmineral zur Verwendung nach einem der vorhergehenden Ansprüche, **dadurch gekennzeichnet, dass** es mit mindestens einer entzündungshemmenden Substanz modifiziert ist.

9. Wechsellagerungstonmineral zur Verwendung nach einem der Ansprüche 1 bis 7, **dadurch gekennzeichnet, dass** es mit mindestens einer weiteren Substanz ausgewählt ist aus einer Gruppe enthaltend Pflanzenextrakte, Fermente, Harze, Antioxidantien, Huminsäuren und deren Salze modifiziert ist.

10. Wechsellagerungstonmineral zur Verwendung nach Anspruch 9, **dadurch gekennzeichnet, dass** das Pflanzenextrakt ausgewählt ist aus einer Gruppe von Extrakten erhältlich aus Grünalgen, Gemüse, Kräutern, Gewürzen und Obst und das Antioxidant ausgewählt ist aus einer Gruppe enthaltend Vitamin C, Vitamin E, Polyphenole und/oder Terpene.

11. Wechsellagerungstonmineral zur Verwendung nach Anspruch 9, **dadurch gekennzeichnet, dass** die Huminsäuren wasserlösliche Fulvosäuren, wasserunlösliche, aber alkalilösliche Huminsäuren und wasser- und alkaliunlösliche Huminstoffe umfassen.

12. Wechsellagerungstonmineral zur Verwendung nach einem der vorhergehenden Ansprüche, **dadurch gekennzeichnet, dass** es mit einer anorganischer Substanz ausgewählt aus der Gruppe der Metallsalze, insbesondere der Calcium-, Natrium- und Magnesiumsalze modifiziert ist.

13. Wechsellagerungstonmineral zur Verwendung nach einem der vorhergehenden Ansprüche, **dadurch gekennzeichnet, dass** die mineralische Verbindung mindestens in einer Einmaldosis mit einer Menge zwischen 1 bis 10 g, bevorzugt 2 bis 8 g, insbesondere bevorzugt 3 bis 5 g bevorzugt bis zu 3 x täglich verabreicht wird.

14. Mit mindestens einer weiteren Substanz ausgewählt aus der Gruppe der Huminsäuren modifiziertes Wechsellagerungstonmineral zur Verwendung nach einem der Ansprüche 1 -7 und 11-13.

## Claims

1. Mixed-layer clay mineral for use as a means of preventing and/or treating chronic inflammatory bowel diseases (CIBD), **characterized in that** the clay mineral is before use milled to a particle size of less than 5 µm and then heat-treated at a temperature between 300°C and 500°C, preferably between 350°C and 450°C, for a period of 1 h to 5 h, preferably 1 h to 3 h.

2. Mixed-layer clay mineral for use according to Claim 1, **characterized in that** the mixed-layer clay mineral comprises a mixed-layer clay mineral consisting of montmorillonite and illite/muscovite.

3. Mixed-layer clay mineral for use according to Claim 1 or 2, **characterized in that** the mixed-layer clay mineral comprises the clay minerals montmorillonite and illite/muscovite in a content of not less than 50% by weight each.

4. Mixed-layer clay mineral for use according to any of the preceding claims, **characterized in that** other clay minerals such as kaolinites and chlorites are present.

5. Mixed-layer clay mineral for use according to any of the preceding claims, **characterized in that** the mineral compound comprises 50-60% by weight of montmorillonite-muscovite ML, 15-25% by weight of illite/muscovite, 5-9% by weight of kaolinite/chlorite, 10-20% by weight of quartz, 1-2% by weight of calcite, 0.9-1.5% by weight of dolomite, 0.9-1.9% by weight of feldspar, 0.9-2.0% by weight of pyrite and 0.6-1.0% by weight of gypsum.

6. Mixed-layer clay mineral for use according to any of the preceding claims, **characterized in that** the matrix has a BET surface area of 50-100 m²/g, preferably 55-65 m²/g, more preferably 60 m²/g.

7. Mixed-layer clay mineral for use according to any of the preceding claims, **characterized in that** the mineral compound is used in a colloidally disperse and/or gel-like form.

8. Mixed-layer clay mineral for use according to any of the preceding claims, **characterized in that** it is modified with at least one anti-inflammatory substance.

9. Mixed-layer clay mineral for use according to any of Claims 1 to 7, **characterized in that** it is modified with at least one further substance selected from a group containing plant extracts, ferments, resins, antioxidants, humic acids and salts thereof.

10. Mixed-layer clay mineral for use according to Claim 9, **characterized in that** the plant extract is selected from a group of extracts obtainable from green algae, vegetables, herbs, spices and fruit and the antioxidant is selected from a group containing vitamin C, vitamin E, polyphenols and/or terpenes.

11. Mixed-layer clay mineral for use according to Claim 9, **characterized in that** the humic acids comprise water-soluble fulvic acids, water-insoluble, but alkali-soluble humic acids, and water-insoluble and alkali-insoluble humic substances.

12. Mixed-layer clay mineral for use according to any of the preceding claims, **characterized in that** it is modified with an inorganic substance selected from the group of metal salts, in particular calcium, sodium and magnesium salts.

13. Mixed-layer clay mineral for use according to any of the preceding claims, **characterized in that** the mineral compound is administered at least in a single dose containing an amount of between 1 to 10 g, preferably 2 to 8 g, more preferably 3 to 5 g, preferably up to 3 times daily.

14. Mixed-layer clay mineral modified with at least one further substance selected from the group of humic acids for use according to any of Claims 1-7 and 11-13.

## Revendications

1. Minéral argileux à couches mixtes destiné à une utilisation en tant qu'agent pour la prévention et/ou le traitement de maladies inflammatoires chroniques de l'intestin (MICI), **caractérisé en ce que** le minéral argileux est broyé avant l'utilisation à une taille de particule inférieure à 5 µm, puis traité thermiquement à une température comprise entre 300 °C et 500 °C, de préférence entre 350 °C et 450 °C, pendant une durée de 1 h à 5 h, de préférence de 1 h à 3 h.

2. Minéral argileux à couches mixtes destiné à une utilisation selon la revendication 1, **caractérisé en ce que** le minéral argileux à couches mixtes comprend un minéral argileux à couches mixtes de montmorillonite et d'illite/muscovite.

3. Minéral argileux à couches mixtes destiné à une utilisation selon la revendication 1 ou 2, **caractérisé en ce que** le minéral argileux à couches mixtes comprend les minéraux argileux montmorillonite et illite/muscovite, chacun à hauteur d'au moins 50 % en poids.

4. Minéral argileux à couches mixtes destiné à une utilisation selon l'une quelconque des revendications précédentes, **caractérisé en ce que** des minéraux argileux supplémentaires tels que la kaolinite et la chlorite sont contenus.

5. Minéral argileux à couches mixtes destiné à une utilisation selon l'une quelconque des revendications précédentes, **caractérisé en ce que** le composé minéral comprend 50 à 60 % en poids de montmorillonite-muscovite-WL, 15 à 25 % en poids d'illite/muscovite, 5 à 9 % en poids de kaolinite/chlorite, 10 à 20 % en poids de quartz, 1 à 2 % en poids de calcite, 0,9 à 1,5 % en poids de dolomite, 0,9 à 1,9 % en poids de feldspath, 0,9 à 2,0 % en poids de pyrite et 0,6 à 1,0 % en poids de gypse.

6. Minéral argileux à couches mixtes destiné à une utilisation selon l'une quelconque des revendications précédentes, **caractérisé en ce que** la matrice présente une surface BET de 50 à 100 m²/g, de préférence de 55 à 65 m²/g, de manière particulièrement préférée de 60 m²/g.

7. Minéral argileux à couches mixtes destiné à une utilisation selon l'une quelconque des revendications précédentes, **caractérisé en ce que** le composé minéral est utilisé sous une forme dispersée colloïdalement et/ou de type gel.

8. Minéral argileux à couches mixtes destiné à une utilisation selon l'une quelconque des revendications précédentes, **caractérisé en ce qu'**il est modifié avec au moins une substance anti-inflammatoire.

9. Minéral argileux à couches mixtes destiné à une utilisation selon l'une quelconque des revendications 1 à 7, **caractérisé en ce qu'**il est modifié avec au moins une substance supplémentaire choisie dans un groupe contenant les extraits végétaux, les ferments, les résines, les antioxydants, les acides humiques et leurs sels.

10. Minéral argileux à couches mixtes destiné à une utilisation selon la revendication 9, **caractérisé en ce que** l'extrait végétal est choisi dans un groupe d'extraits pouvant être obtenus à partir d'algues vertes, de légumes, d'herbes, d'épices et de fruits, et l'antioxydant est choisi dans un groupe contenant la vitamine C, la vitamine E, les polyphénols et/ou les terpènes.

11. Minéral argileux à couches mixtes destiné à une utilisation selon la revendication 9, **caractérisé en ce que** les acides humiques comprennent des acides fulviques solubles dans l'eau, des acides humiques insolubles dans l'eau, mais solubles dans les alcalis, et des matières humiques insolubles dans l'eau et dans les alcalis.

12. Minéral argileux à couches mixtes destiné à une utilisation selon l'une quelconque des revendications précédentes, **caractérisé en ce qu'**il est modifié avec une substance inorganique choisie dans le groupe des sels de métaux, notamment des sels de calcium, sodium et magnésium.

13. Minéral argileux à couches mixtes destiné à une utilisation selon l'une quelconque des revendications précédentes, **caractérisé en ce que** le composé minéral est administré au moins en une dose unique en une quantité de 1 à 10 g, de préférence de 2 à 8 g, de manière particulièrement préférée de 3 à 5 g, de préférence jusqu'à 3 x par jour.

14. Minéral argileux à couches mixtes modifié avec au moins une substance supplémentaire choisie dans le groupe des acides humiques, destiné à une utilisation selon l'une quelconque des revendications 1 à 7 et 11 à 13.
